# EUROPEAN PATENT APPLICATION

(11) **EP 4 474 393 A1**
(43) Date of publication of application: **11.12.2024**
(21) Application number: 23204223.4
(22) Date of filing: 17.10.2023
(51) Int. Cl.: C07K 16/10, A61P 31/18

(54) **BROADLY NEUTRALIZING ANTIBODIES TARGETING THE CD4 BINDING SITE ON HIV ENV**

(30) Priority: 09.06.2023 EP 23178486
(71) Applicant: Universität zu Köln, 50923 Köln (DE)
(72) Inventor: KLEIN, Florian, 50933 Köln (DE); GIESELMANN, Lutz, 50935 Köln (DE)
(74) Representative: Klöckner, Christoph

(57) **Abstract**

The present disclosure relates to monoclonal human antibodies or binding fragments thereof which are directed against the CD4 binding site of the human immunodeficiency virus HIV-1, a pharmaceutical composition comprising such monoclonal human antibodies or binding fragments thereof, a kit comprising such antibodies or binding fragments thereof, and the monoclonal antibodies or binding fragments thereof and the pharmaceutical composition and the kit for use as a medicament, and in the treatment or prevention of a disease caused by the human immunodeficiency virus HIV-1.

## Description

### Technical field

The present invention relates to monoclonal human antibodies or binding fragments thereof which are directed against the CD4 binding site of the human immunodeficiency virus HIV-1, a pharmaceutical composition comprising such monoclonal human antibodies or binding fragments thereof, a kit comprising such antibodies or binding fragments thereof, and the monoclonal antibodies or binding fragments thereof and the pharmaceutical composition and the kit for use as a medicament, and in the treatment, cure or prevention of a disease caused by the human immunodeficiency virus HIV-1.

### Background of the Invention

Despite the widespread use of effective antiretroviral therapy (ART), the combat against the global HIV-1 epidemic still remains a public health priority. Highly potent and broadly neutralizing anti-HIV-1 antibodies (bNAbs) represent promising and powerful tools that open unprecedented opportunities for alternative treatment and prevention strategies. In recent years, potent bNAbs have been isolated from HIV-1-infected donors that bind to distinct vulnerable epitopes on the HIV-1 envelope (Env) trimer. These epitopes encompass the CD4 binding site (CD4bs), the V1/V2 loop, the V3 loop glycan patch, the membrane-proximal external region, the fusion peptide, the silent face and the interface between the gp120 and gp41 Env subunits.

Among these sites, the CD4bs is of particular interest because CD4 serves as the primary receptor for viral entry. BNAbs that are encoded by the immunoglobulin heavy chain gene segment IGHV1-2 often mimic the CD4 contact residues for the viral envelope protein, display high levels of somatic hypermutation, and rely on a five amino acid light chain complementarity determining region (CDRL3). These antibodies are also referred to as VRC01-class antibodies and comprise the majority of the most potent and broadest CD4bs bNAbs identified to date. Due to their remarkable antiviral capacities, several CD4bs bNAbs have entered advanced clinical. Representative members of the VRC01-class include VRC01, VRC07, VRC07-523-LS, 3BNC117, NIH45-46, N6, and N49-P7.

Other CD4bs bNAbs are encoded by the immunoglobulin heavy chain gene segment IGHV1-46 and do not require a five amino acid CDRL3. Representative members of this antibody class are 8ANC131, CH235, and 1B2530. These antibodies mostly display lower potencies and breadth when compared to VRC01-class antibodies. One exception is the recently identified bNAb 1-18 that exhibits high levels of potency and breadth against large multiclade HIV-1 pseudovirus reference panels.

In pre-clinical animal models bNAbs have demonstrated robust protection from infection even at low levels of serum concentrations and have shown favorable safety and pharmacokinetic profiles. Moreover, administration of bNAbs in HIV-1-infected individuals has led to prolonged suppression of viremia and delayed viral rebound following analytical treatment interruption (ATI). While the recent Antibody Mediated Prevention (AMP) efficacy trials using the CD4bs bNAb VRC01 did not demonstrate overall efficacy in preventing HIV-1 infection, subgroup analyses showed efficacy of VRC01 on prevention of infection with VRC01-sensitive strains (IC80 < 1 µg/ml). Therefore, the identification and development of highly potent CD4bs bNAbs is of utmost relevance for HIV-1 prevention. These bNAbs need to have enhanced neutralization potency, breadth and restricted viral escape pathways. Therefore, there remains a strong demand for the isolation of novel CD4bs bNAbs that exceed the antiviral activity of known anti-HIV-1 bNAbs.

The present invention aims to provide novel human monoclonal antibodies targeting HIV-1 that exhibit remarkable levels of breadth and potency against various virus strains and subtypes.

Moreover, it is a further objective of this invention to provide novel human monoclonal antibodies directed against HIV-1 that limit the emergence of viral escape variants and remain effective against such variants.

### Summary of the Invention

These objects have been solved by the aspects of the present invention as specified hereinafter.

According to the first aspect of the present invention, an antibody or antigen-binding fragment thereof directed against the CD4 binding site of the human immunodeficiency virus HIV-1 is provided, wherein the antibody or antigen-binding fragment thereof comprises a combination of the heavy chain CDR1 to CDR3 and the light chain CDR1 to CDR3 amino acid sequence of one antibody from the group comprising **04_A06** (having a CDR-H1 amino acid sequence of SEQ ID No. 1, a CDR-H2 amino acid sequence of SEQ ID No. 2, a CDR-H3 amino acid sequence of SEQ ID No. 3, a CDR-L1 amino acid sequence of SEQ ID No. 4, a CDR-L2 amino acid sequence of SEQ ID No. 5, and a CDR-L3 amino acid sequence of SEQ ID No. 6), **01_D03** (having a CDR-H1 amino acid sequence of SEQ ID No. 7, a CDR-H2 amino acid sequence of SEQ ID No. 8, a CDR-H3 amino acid sequence of SEQ ID No. 9, a CDR-L1 amino acid sequence of SEQ ID No. 10, a CDR-L2 amino acid sequence of SEQ ID No. 11, and a CDR-L3 amino acid sequence of SEQ ID No. 12), **04_B09** (having a CDR-H1 amino acid sequence of SEQ ID No. 13, a CDR-H2 amino acid sequence of SEQ ID No. 14, a CDR-H3 amino acid sequence of SEQ ID No. 15, a CDR-L1 amino acid sequence of SEQ ID No. 16, a CDR-L2 amino acid sequence of SEQ ID No. 17, and a CDR-L3 amino acid sequence of SEQ ID No. 18), **05_C07** (having a CDR-H1 amino acid sequence of SEQ ID No. 19, a CDR-H2 amino acid sequence of SEQ ID No. 20, a CDR-H3 amino acid sequence of SEQ ID No. 21, a CDR-L1 amino acid sequence of SEQ ID No. 22, a CDR-L2 amino acid sequence of SEQ ID No. 23, and a CDR-L3 amino acid sequence of SEQ ID No. 24), and **01_B01** (having a CDR-H1 amino acid sequence of SEQ ID No. 25, a CDR-H2 amino acid sequence of SEQ ID No. 26, a CDR-H3 amino acid sequence of SEQ ID No. 27, a CDR-L1 amino acid sequence of SEQ ID No. 28, a CDR-L2 amino acid sequence of SEQ ID No. 29, and a CDR-L3 amino acid sequence of SEQ ID No. 30).

According to a preferred embodiment of the first aspect of the invention, the antibody or antigen-binding fragment thereof comprises a combination of the variable region heavy chain amino acid sequence and of the variable region light chain amino acid sequence of one antibody selected from the group comprising **04_A06** (consisting of the heavy chain amino acid sequence of SEQ ID No. 31 and the light chain amino acid sequence of SEQ ID No. 32), **01_D03** (consisting of the heavy chain amino acid sequence of SEQ ID No. 33 and the light chain amino acid sequence of SEQ ID No. 34), **04_B09** (consisting of the heavy chain amino acid sequence of SEQ ID No. 35 and the light chain amino acid sequence of SEQ ID No. 36), **05_C07** (consisting of the heavy chain amino acid sequence of SEQ ID No. 37 and the light chain amino acid sequence of SEQ ID No. 38), and **01_B01** (consisting of the heavy chain amino acid sequence of SEQ ID No. 39 and the light chain amino acid sequence of SEQ ID No. 40).

According to a preferred embodiment of the first aspect of the invention or of the preceding embodiment of the first aspect of the invention, the amino acid sequences comprised in the antibody or the antigen-binding fragment thereof are of one antibody selected from the group comprising 04_A06, 01_D03, 04_B09 and 05_C07, preferably of one antibody from the group comprising 04_A06, 01_D03, and 04_B09, more preferably of one antibody from the group comprising 04_A06 and 01_D03, particularly preferably of the antibody 04_A06.

According to the second aspect of the present invention, a pharmaceutical composition is provided comprising an antibody or antigen-binding fragment thereof according to the first aspect of the present invention, and at least one pharmaceutically acceptable excipient.

According to a preferred embodiment of the second aspect of the invention, the pharmaceutical composition is a vaccination composition for a human subject.

According to the third aspect of the present invention, a kit is provided comprising an antibody or antigen-binding fragment thereof according to the first aspect of the present invention and a container.

According to the fourth aspect of the present invention, an antibody or antigen-binding fragment thereof according to the first aspect of the present invention, a pharmaceutical composition according to the second aspect of the present invention, or a kit according to the third aspect of the present invention is provided for use as a medicament.

According to the fifth aspect of the present invention, an antibody or antigen-binding fragment thereof according to the first aspect of the present invention, a pharmaceutical composition according to the second aspect of the present invention, or a kit according to the third aspect of the present invention is provided for use in the treatment or prevention of an infection with the human immunodeficiency virus HIV-1 in mammalian subjects.

According to the sixth aspect of the present invention, an antibody or antigen-binding fragment thereof according to the first aspect of the present invention, a pharmaceutical composition according to the second aspect of the present invention, or a kit according to the third aspect of the present invention is provided for use in the treatment or prevention of a disease caused by the human immunodeficiency virus HIV-1 in mammalian subjects.

In a preferred embodiment of the fifth or sixth aspect of the present invention, the use is in human subjects.

In one preferred embodiment of the fifth or sixth aspect of the present invention, the use is in in the treatment or prevention of acquired immune deficiency syndrome (AIDS).

According to the seventh aspect of the present invention, a nucleic acid is provided encoding an antibody or antigen-binding fragment thereof according to the first aspect of the present invention.

According to the eighth aspect of the present invention, an expression vector comprising the nucleic acid of the seventh aspect of the present invention is provided in functional association with an expression control sequence.

According to the ninth aspect of the present invention, a host cell is provided comprising a nucleic acid according to the seventh aspect of the present invention or the expression vector according to the eighth aspect of the present invention.

According to the tenth aspect of the present invention, a method of production of an antibody or antigen-binding fragment thereof according to the first aspect of the present invention is provided, comprising cultivating the host cell of the ninth aspect of the present invention under conditions allowing expression of the antibody or antigen-binding fragment thereof, and recovering the antibody or antigen-binding fragment thereof.

### Description of Figures

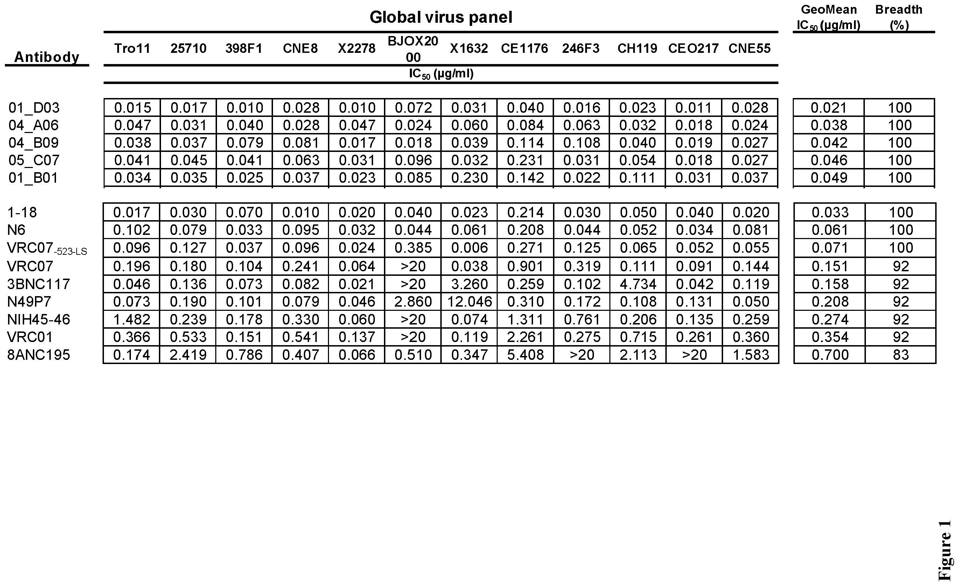

Figure 1 shows the neutralizing activity of antibodies of the present invention against a panel of 12 global reference pseudovirus strains of HIV-1 with different envelope amino acid sequences (as described in de Camp et al., J Virol. 2014 Mar; 88(5): 2489-2507) when tested in the TZM-bl cell pseudovirus neutralization assay (see below in the Examples section); antibodies 04_A06 and 01_D03 as described herein neutralize 100% (12/12) strains of the "Global Panel" and display high levels of potency.

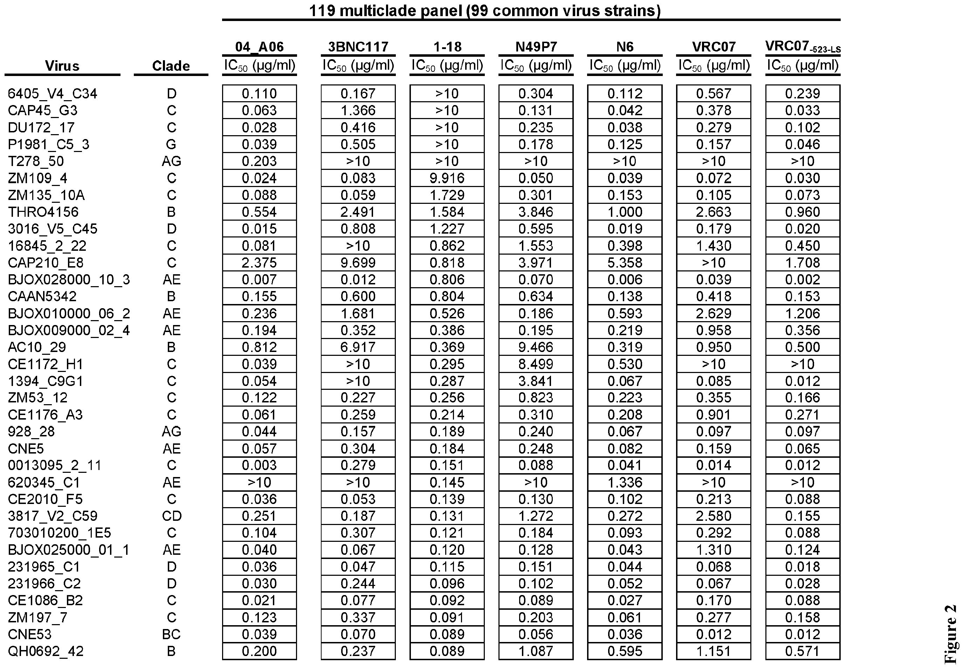

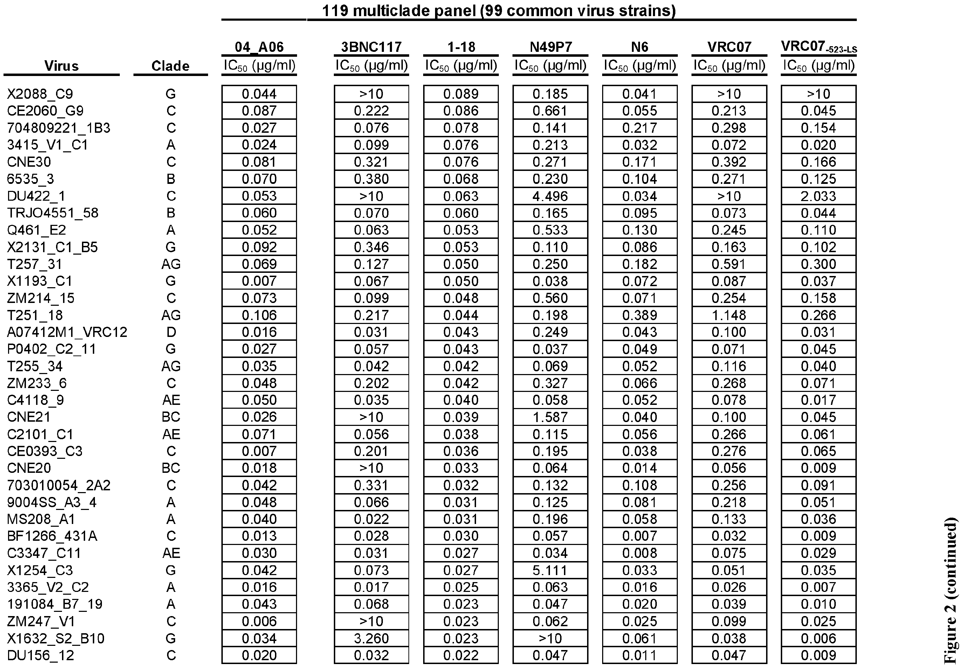

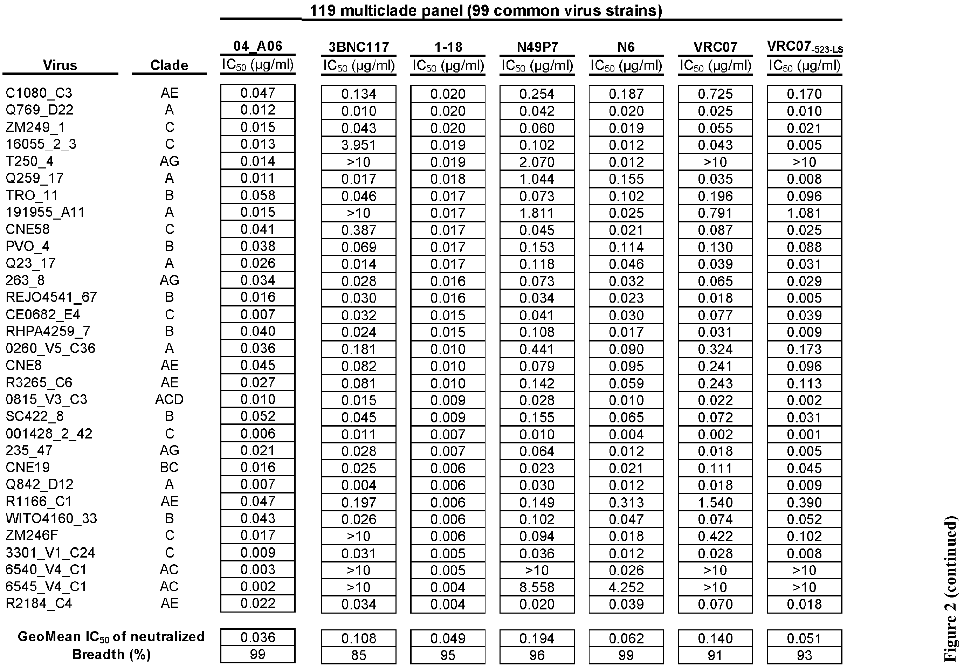

Figure 2 shows the neutralizing activity (IC₅₀) of antibody 04_A06 of the present invention against a multiclade panel of 119 pseudoviruses with different HIV-1 envelope amino acid sequences when tested in the TZM-bl cell pseudovirus neutralization assay (as described in Schoofs et al., Immunity, 2019 Jun 18;50(6):1513-1529.e9); data for antibody 04_A06 compared to the most potent and broadest CD4bs bNAbs of the prior art (3BNC117, 1-18, N49P7, N6, VRC07, and VRC07_{-523-LS}) are shown; for presentation in this figure, 99 pseudovirus strains from the 119 multiclade panel for which neutralization data of reference antibodies was published were selected; neutralization data of the reference bNAbs were retrieved from the antibody neutralization database CATNAP (Yoon et al., Nucleic Acids Research, Volume 43, Issue W1, 1 July 2015, Pages W213-W219, https://doi.org/10.1093/nar/gkv404).

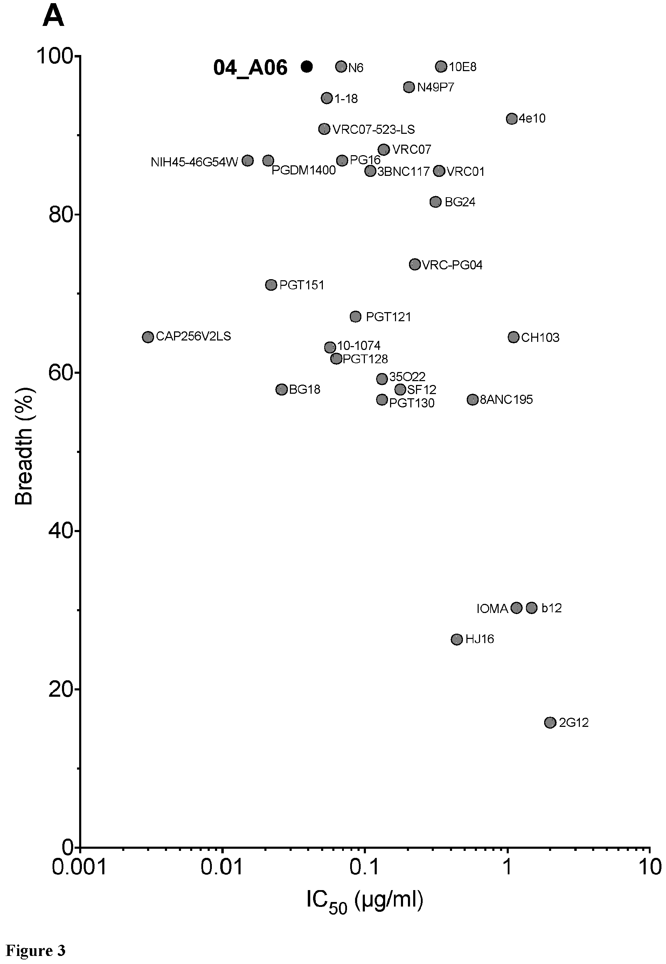

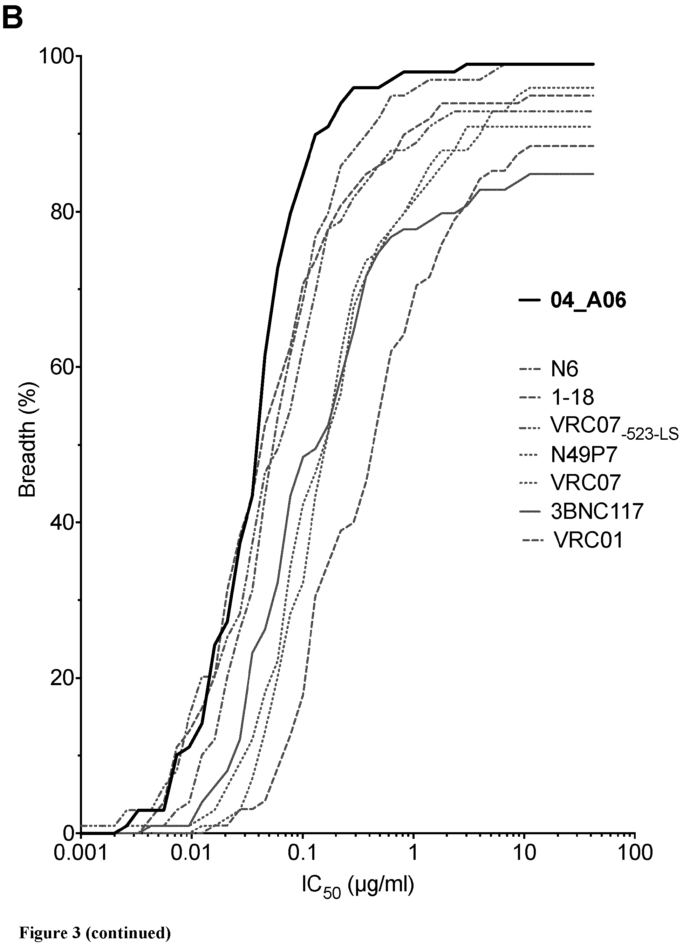

Figure 3 shows the neutralizing activity (IC₅₀ and breadth) of the antibody 04_A06 of the present invention compared to anti-HIV-1 antibodies of the prior art against a multiclade panel of 119 pseudoviruses with different HIV-1 envelope amino acid sequences when tested in the TZM-bl cell pseudovirus neutralization assay (as in Figure 2, and described in Schoofs et al., Immunity, 2019 Jun 18;50(6):1513-1529.e9); for presentation in this figure, 76 pseudovirus strains (A) or 99 pseudovirus strains (**B**) from the 119 multiclade panel for which neutralization data of reference antibodies was published were selected; (**A**) shows the neutralizing activity of 04_A06 compared to other anti-HIV-1 bNAbs that target a large variety of different known epitopes, (**B**) presents the neutralizing activity of antibody 04_A06 compared to the most potent and broadest anti-HIV-1 bNAbs of the prior art; neutralization data of the reference bNAbs were retrieved from the antibody neutralization database CATNAP (Yoon et al., 2015, *supra*)*.*

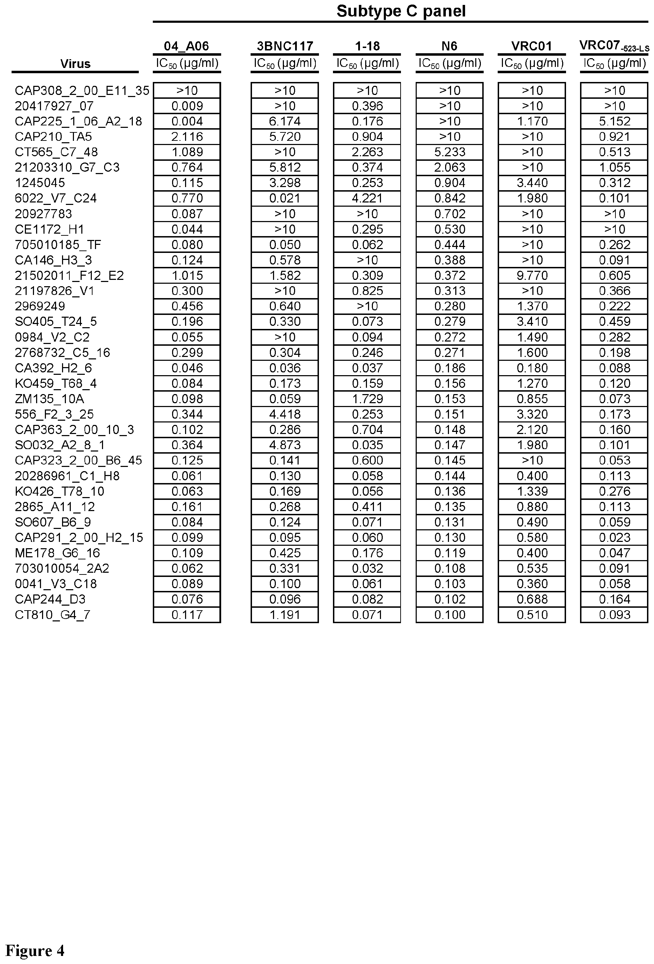

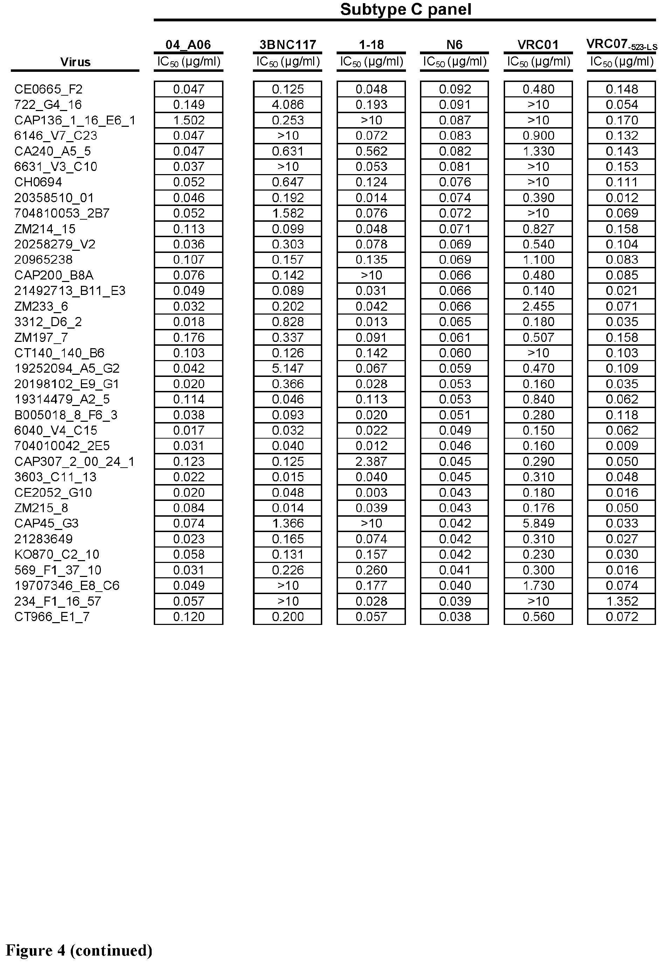

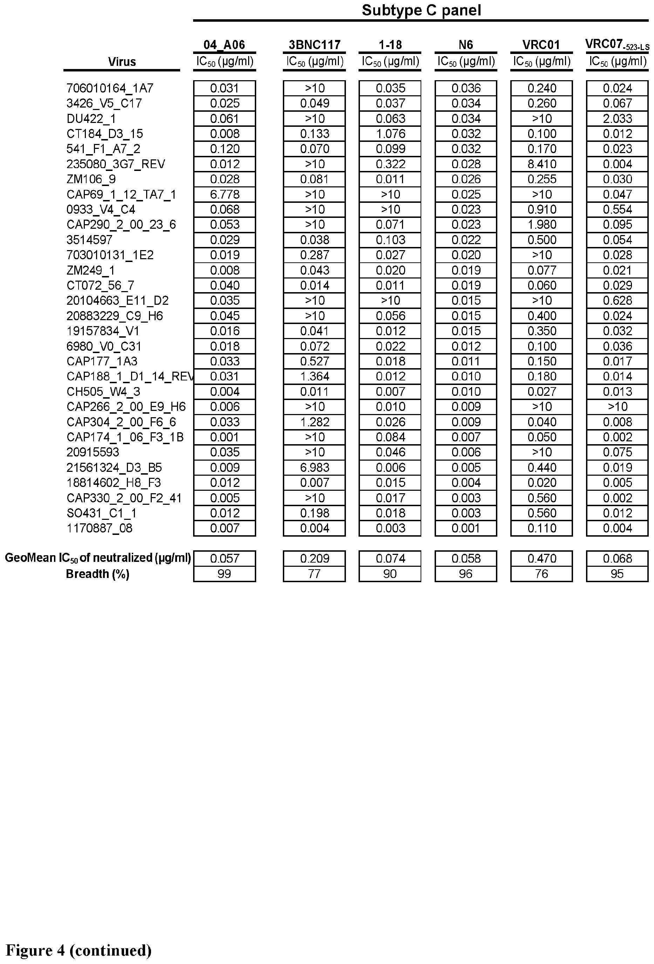

Figure 4 shows the neutralizing activity (IC₅₀) of the antibody 04_A06 of the present invention against a multiclade panel of 100 clade C HIV-1 pseudoviruses (Subtype C panel) with different HIV-1 envelope amino acid sequences when tested in the TZM-bl cell pseudovirus neutralization assay (as described in Hraber et al., J Virol., 2017 Sep 12;91(19):e00991-17); data for antibody 04_A06 compared to the most potent and broadest CD4bs bNAbs of the prior art (3BNC117, 1-18, N6, VRC01, and VRC07-523-LS) are shown; neutralization data of the reference bNAbs were retrieved from the antibody neutralization database CATNAP (Yoon et al., 2015, *supra).*

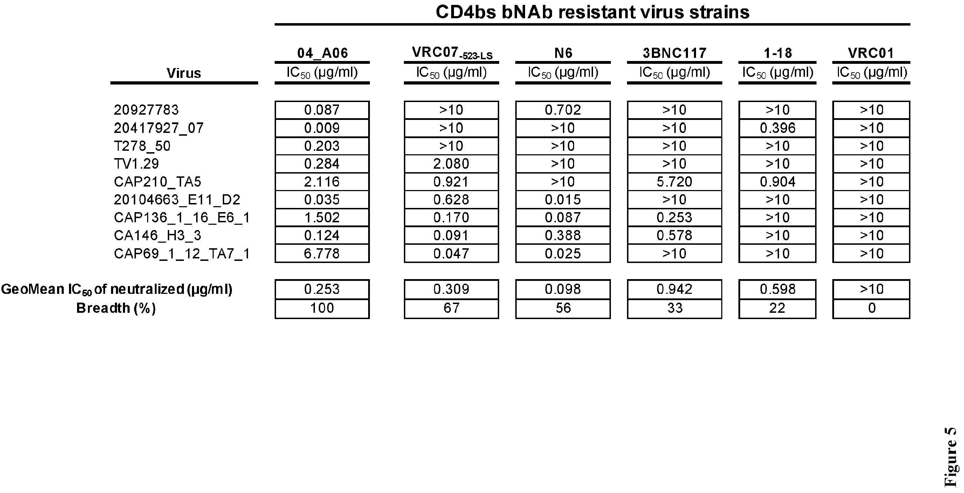

Figure 5 shows the neutralizing activity (IC₅₀) of the antibody 04_A06 of the present invention against a panel of 9 pseudoviruses with different HIV-1 envelope amino acid sequences when tested in the TZM-bl cell pseudovirus neutralization assay; the pseudoviruses of this panel are known to display extraordinary resistance against CD4bs bNAbs; viral strains of this panel are constituents of the 119 multiclade and Subtype C pseudovirus panel (as described in Schoofs et al., Immunity, 2019 Jun 18;50(6):1513-1529.e9; and Hraber et al., J Virol., 2017 Sep 12;91(19):e00991-17); data for antibody 04_A06 compared to the most potent and broadest CD4bs bNAbs described to date (VRC07_{-523-LS}, N6, 3BNC117, 1-18, VRC01) are shown; neutralization data of the reference bNAbs were retrieved from the antibody neutralization database CATNAP (Yoon et al., 2015, *supra).*

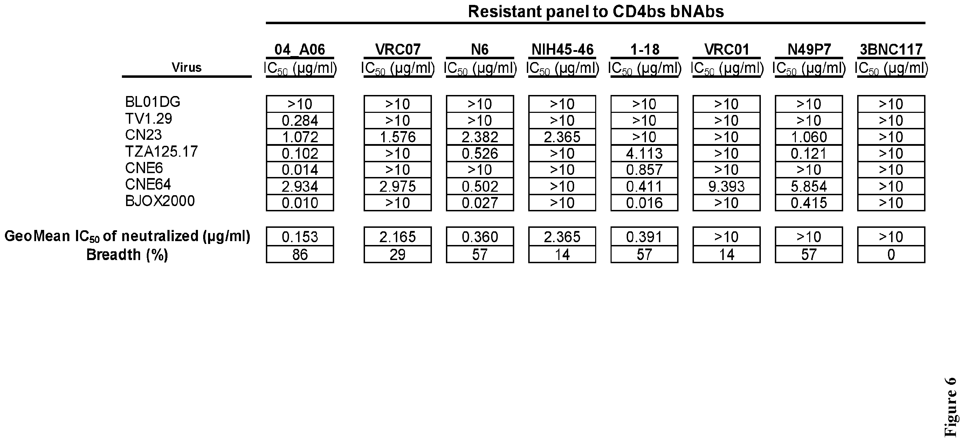

Figure 6 shows the neutralizing activity (IC₅₀) of the antibody 04_A06 of the present invention against a panel of 7 pseudoviruses with different HIV-1 envelope amino acid sequences when tested in the TZM-bl cell pseudovirus neutralization assay; tested virus strains were selected from a reference pseudovirus panel that is known as "Resistant panel to CD4bs bNAbs" and is described in Zhou et al., PLoS Pathog. 2019 Jun 13;15(6):e1007819. doi: 10.1371/journal.ppat.1007819. PMID: 31194843; PMCID: PMC6592578 2017 Sep 12;91(19):e00991-17; the pseudoviruses of this published reference panel display extraordinary resistance against CD4bs bNAbs; data for antibody 04_A06 compared to the most potent and broadest CD4bs bNAbs of the prior art (VRC07, N6, NIH45-46, 1-18, VRC01, N49P7, 3BNC117) are shown; all antibodies were tested in duplicates in independent assays.

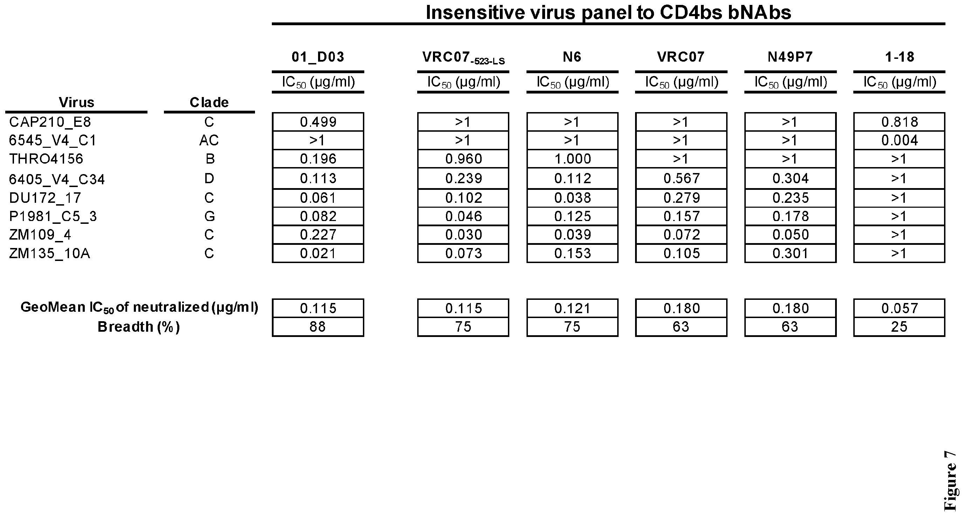

Figure 7 shows the neutralizing activity (IC₅₀) of the antibody 01_D03 of the present invention against a panel of 8 pseudoviruses with different HIV-1 envelope amino acid sequences when tested in the TZM-bl cell pseudovirus neutralization assay; viral strains of this panel are constituents of the 119 multiclade (as described in Schoofs et al., Immunity, 2019 Jun 18;50(6):1513-1529.e9); selected pseudoviruses of this panel are insensitive against neutralization by CD4bs bNAbs; insensitivity against antibody neutralization was defined by an IC₅₀ > 1 µg/ml; data for antibody 01_D03 compared to the most potent and broadest CD4bs bNAbs of the prior art (VRC07-523-LS, N6, VRC07, N49P7, 1-18) are shown; all antibodies were tested in duplicates in independent assays. neutralization data of the reference bNAbs were retrieved from the antibody neutralization database CATNAP (Yoon et al., 2015, *supra*)*.*

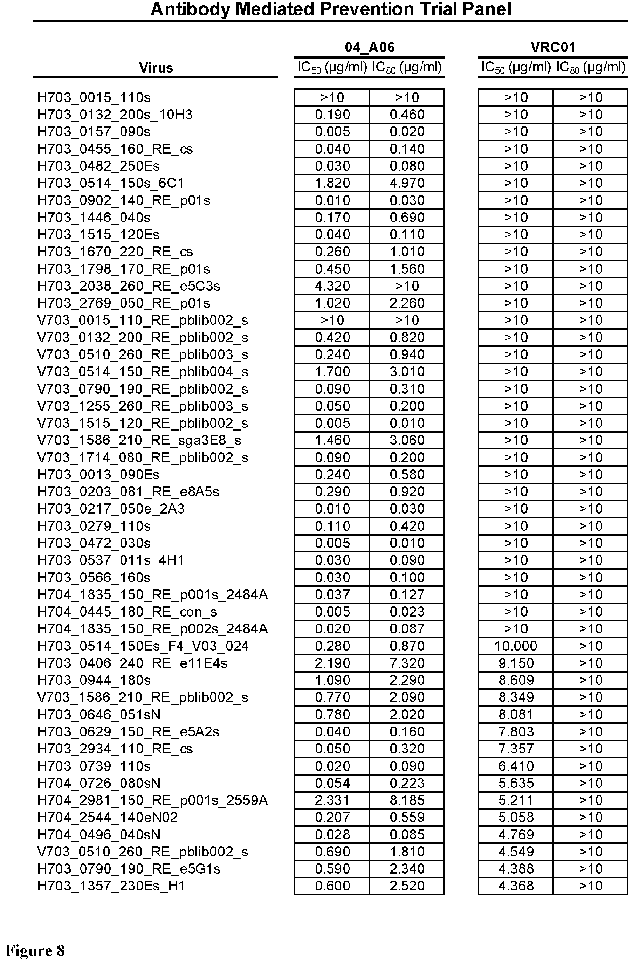

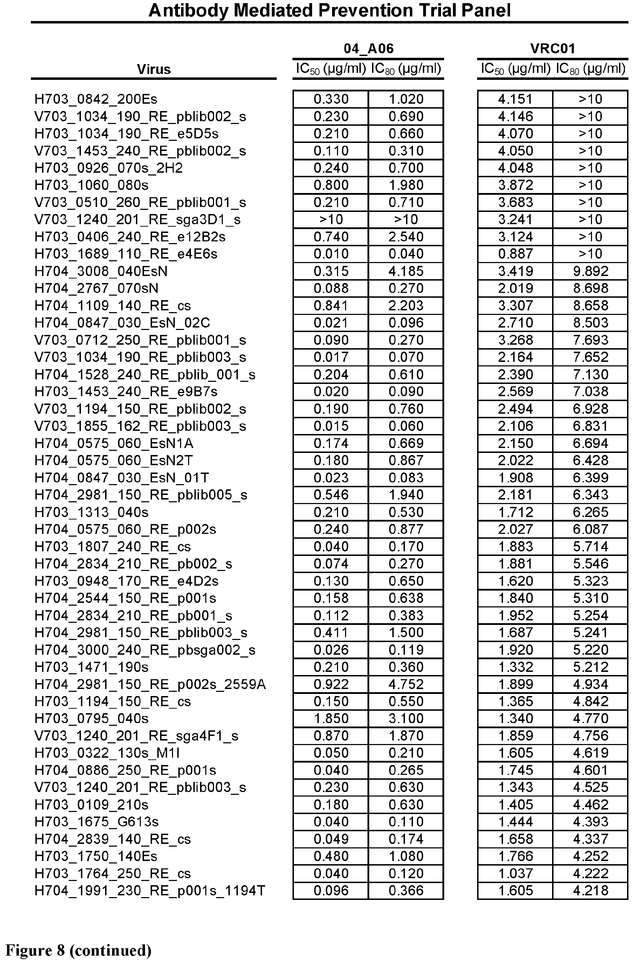

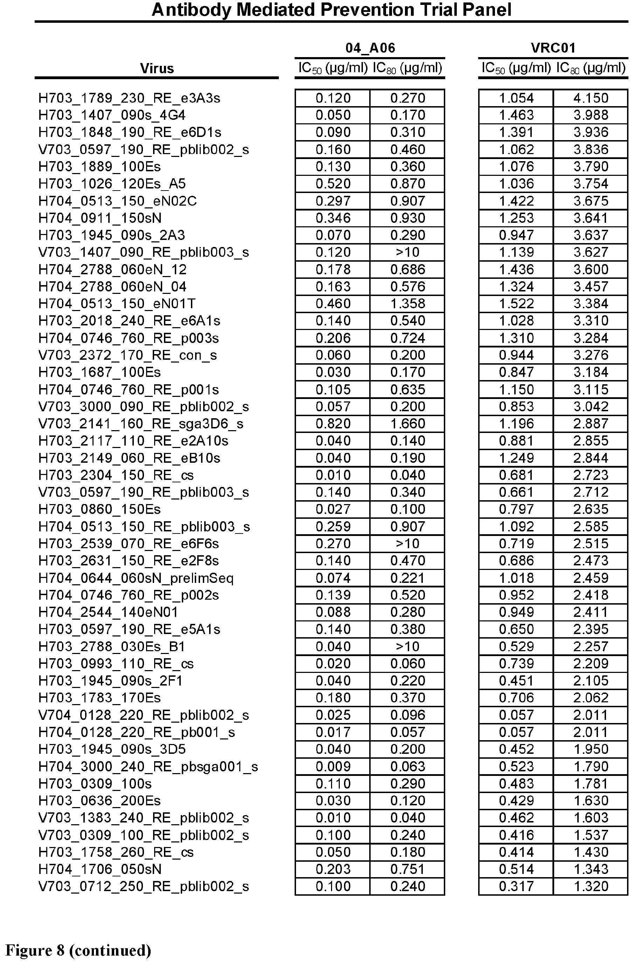

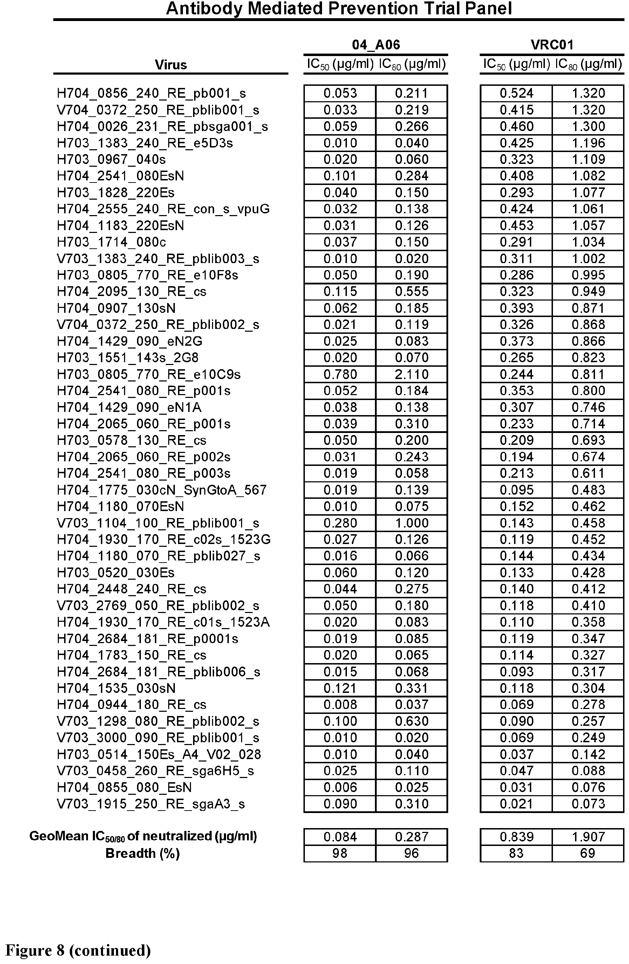

Figure 8 shows the neutralizing activity (IC₅₀ and IC₈₀) of the antibody 04_A06 of the present invention against a panel of 185 pseudoviruses with different HIV-1 envelope amino acid sequences when tested in the TZM-bl cell pseudovirus neutralization assay; viral strains of this pseudovirus panel were generated from virus sequences obtained from the Antibody Mediated Prevention Trials (as described in Corey L, Two Randomized Trials of Neutralizing Antibodies to Prevent HIV-1 Acquisition. N Engl J Med. 2021 Mar 18;384(11):1003-1014. doi: 10.1056/NEJMoa2031738. PMID: 33730454; PMCID: PMC8189692); data for antibody 04_A06 compared to reference bNAb VRC01 is shown; neutralization data for other reference bNAbs was not publicly available; neutralization data of the reference bNAb VRC01 was retrieved from Corey L, Two Randomized Trials of Neutralizing Antibodies to Prevent HIV-1 Acquisition. N Engl J Med. 2021 Mar 18;384(11):1003-1014. Doi: 10.1056/NEJMoa2031738. PMID: 33730454; PMCID: PMC8189692.

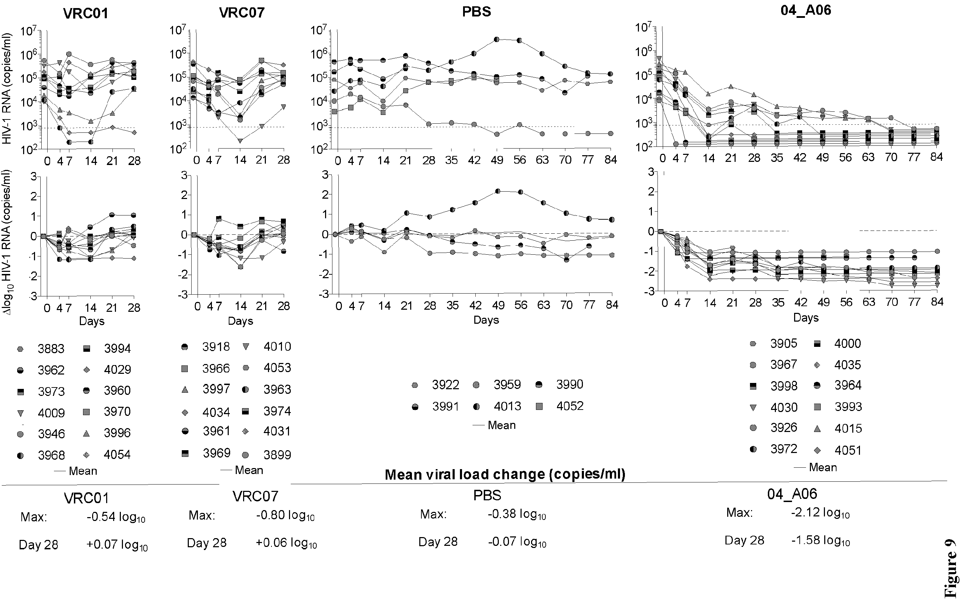

Figure 9 shows HIV-1 RNA plasma copy numbers (top) and their log₁₀ change (bottom, compared to baseline (HIV-1 RNA plasma copy number of day -1)) in humanized mice infected with HIV-1_{YU2} (NL4-3 virus with envelope gene substituted for that of YU2); mice were treated subcutaneously with a 1 mg loading dose per antibody, followed by subcutaneous injections of 0.5 mg per antibody every 3-4 days; mice treated with the known CD4bs antibodies VRC01 and VRC07 show a transient reduction of viremia, followed by rapid viral rebound; in contrast, mice treated with antibody 04_A06 of the invention alone show a persistent reduction of viremia throughout the treatment period of 84 days; black lines indicate mean log₁₀ change.

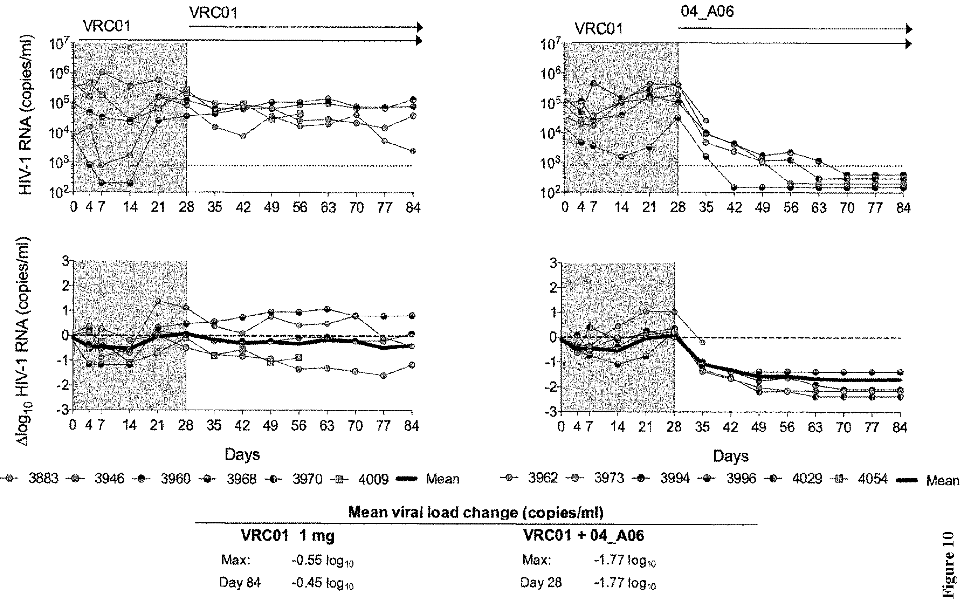

Figure 10 shows HIV-1 RNA plasma copy numbers (top) and their log₁₀ change (bottom, compared to baseline (HIV-1 RNA plasma copy number at day 28)) in humanized mice infected with HIV-1_{YU2} (NL4-3/YU2) that were pre-treated with the CD4 binding site antibody VRC01 (as shown in Figure 8); after four weeks of treatment, antibody 04_A06 was added to the previous treatment regimen that was continued throughout; 04_A06 was administered subcutaneously with a 1 mg loading dose, followed by 0.5 mg every 3 days to 4 days; despite viral rebound after pre-treatment with the CD4 binding site antibody VRC01, treatment with antibody 04_A06 resulted in maintained viral suppression in all treated animals.

### Detailed Description of the Invention

The present inventors have dedicated themselves to solving the problem of the present invention and were successful to find novel and useful human monoclonal antibodies against HIV-1 which overcome the disadvantages and shortcomings of known antibodies.

Herein, the inventors describe novel CD4 binding site antibodies that exceed the potency and breadth of known CD4bs bNAbs such as antibody 1-18 as disclosed in WO 2021/110764 A1 or others. These properties are unprecedented and open new possibilities for antibody-based therapies and prevention of HIV-1 associated disease.

Thus, the present invention provides in a first aspect an antibody or antigen-binding fragment thereof directed against the CD4 binding site of the human immunodeficiency virus HIV-1, wherein the antibody or antigen-binding fragment thereof comprises a combination of the heavy chain CDR1 to CDR3 and the light chain CDR1 to CDR3 amino acid sequence of one antibody from the group comprising **04_A06** (having a CDR-H1 amino acid sequence of SEQ ID No. 1, a CDR-H2 amino acid sequence of SEQ ID No. 2, a CDR-H3 amino acid sequence of SEQ ID No. 3, a CDR-L1 amino acid sequence of SEQ ID No. 4, a CDR-L2 amino acid sequence of SEQ ID No. 5, and a CDR-L3 amino acid sequence of SEQ ID No. 6), **01_D03** (having a CDR-H1 amino acid sequence of SEQ ID No. 7, a CDR-H2 amino acid sequence of SEQ ID No. 8, a CDR-H3 amino acid sequence of SEQ ID No. 9, a CDR-L1 amino acid sequence of SEQ ID No. 10, a CDR-L2 amino acid sequence of SEQ ID No. 11, and a CDR-L3 amino acid sequence of SEQ ID No. 12), **04_B09** (having a CDR-H1 amino acid sequence of SEQ ID No. 13, a CDR-H2 amino acid sequence of SEQ ID No. 14, a CDR-H3 amino acid sequence of SEQ ID No. 15, a CDR-L1 amino acid sequence of SEQ ID No. 16, a CDR-L2 amino acid sequence of SEQ ID No. 17, and a CDR-L3 amino acid sequence of SEQ ID No. 18), **05_C07** (having a CDR-H1 amino acid sequence of SEQ ID No. 19, a CDR-H2 amino acid sequence of SEQ ID No. 20, a CDR-H3 amino acid sequence of SEQ ID No. 21, a CDR-L1 amino acid sequence of SEQ ID No. 22, a CDR-L2 amino acid sequence of SEQ ID No. 23, and a CDR-L3 amino acid sequence of SEQ ID No. 24), and **01_B01** (having a CDR-H1 amino acid sequence of SEQ ID No. 25, a CDR-H2 amino acid sequence of SEQ ID No. 26, a CDR-H3 amino acid sequence of SEQ ID No. 27, a CDR-L1 amino acid sequence of SEQ ID No. 28, a CDR-L2 amino acid sequence of SEQ ID No. 29, and a CDR-L3 amino acid sequence of SEQ ID No. 30).

Within the context of the present invention, the antibodies, which have been generated and described herein, may be used and claimed as the complete monoclonal human antibody or as any functional or binding fragment thereof. Preferably, the monoclonal human antibody or any kind of functional or antigen-binding fragment thereof should at least comprise the complementarity determining regions (CDR) 1 to 3 of the heavy chain and CDR 1 to 3 of the light chain of the human monoclonal antibody.

The CDR regions of the antibody sequences described herein are preferably defined according to the numbering scheme of IMGT which is an adaptation of the numbering scheme of Chothia (ImMunoGeneTics information system^{®}; Lefranc et al., NAR 27: 209-212 (1999); http://www.imgt.org ).

According to the present invention, the CDR sequences of the light and heavy chain variable region sequences of the antibodies and antigen-binding fragments thereof described herein are as follows:

| **Source** | **CDR1** | **SEQ ID CDR1** | **CDR2** | **SEQ ID CDR2** | **CDR3** | **SEQ ID CDR3** |
|---|---|---|---|---|---|---|
| Heavy chain **04_A06** | DSFTVYRPRL SAYFIGEFN | 1 | VNIFR GAV | 2 | AWDKNVDD NPWRLDS | 3 |
| Light chain **04_A06** | QDVGWA | 4 | TAH | 5 | QVFDS* | 6 |
| Heavy chain **01_D03** | GYSFRDYR | 7 | MQPR YGAV | 8 | VTHKLYDGE DASDLTWR LDP | 9 |
| Light chain **01_D03** | QDITNA | 10 | DGS | 11 | QHYEF** | 12 |
| Heavy chain **04_B09** | AYFIGEFN | 13 | VNIFR GAV | 14 | AWDKNVDD NPWRLDS | 15 |
| Light chain **04_B09** | QDVGWA | 16 | TTS | 17 | QVFES* | 18 |
| Heavy chain **05_C07** | GYTFRDYR | 19 | LHPAY GAV | 20 | VTHKLYDGE DASDLTWR LDP | 21 |
| Light chain **05_C07** | QDITNA | 22 | DGS | 23 | QHYEF** | 24 |
| Heavy chain **01_B01** | GYSFRDYR | 25 | IQPRY GAV | 26 | VTHKLYDGE DASDLAWR LDP | 27 |
| Light chain **01_B01** | QDITNA | 28 | DGS | 29 | QHYEF** | 30 |

| | | | | | | |
|---|---|---|---|---|---|---|
| * The CDRL3 of antibodies 04_A06 and 04_809 could not precisely be determined using the IMGT software. The reason is assumed to lie in the fact that the specific sequences did not fulfil the criteria of the numbering scheme and CDR3 sequence definition of IMGT. CDRL3 sequences of 04_A06 and 04_809 as stated are defined herein on the assumption of an amino acid substitution in the J-region motif (F/W-G-X-G) from F-**G**-P-G to F-**A**-P-G and in comparison to gene sequences from other IGHV1-2 and IGKV1-33 encoded CD4bs bNAbs. ** The CDRL3 of antibody 01_D03, 05_C07 and 01_801 could not precisely be determined using the IMGT software. The reason is assumed to lie in the fact that the specific sequence did not fulfil the criteria of the numbering scheme and CDR3 sequence definition of IMGT. CDRL3 sequence of 01_D03 as stated is defined herein on the assumption of an amino acid substitution in the J-region motif (F/W-G-X-G) from F-**G-P-G** to F**-P-P-V** and in comparison to gene sequences from other IGHV1-2 and IGKV1-33 encoded CD4bs bNAbs. CDRL3 sequences of 05_C07 and 01_801 as stated are defined herein on the assumption of an amino acid substitution in the J-region motif (F/W-G-X-G) from F-**G-P-G** to F**-P-P-A** and in comparison to gene sequences from other IGHV1-2 and IGKV1-33 encoded CD4bs bNAbs. | | | | | | |

Also, one preferred embodiment of the first aspect of the present invention provides antibodies or antigen-binding fragments directed against the CD4 binding site of HIV-1, wherein the antibody or antigen-binding fragment thereof comprises the combination of the variable region heavy chain amino acid sequence and of the variable region light chain amino acid sequence of one antibody selected from the group comprising **04_A06** (consisting of the heavy chain amino acid sequence of SEQ ID No. 31 and the light chain amino acid sequence of SEQ ID No. 32), **01_D03** (consisting of the heavy chain amino acid sequence of SEQ ID No. 33 and the light chain amino acid sequence of SEQ ID No. 34), **04_B09** (consisting of the heavy chain amino acid sequence of SEQ ID No. 35 and the light chain amino acid sequence of SEQ ID No. 36), **05_C07** (consisting of the heavy chain amino acid sequence of SEQ ID No. 37 and the light chain amino acid sequence of SEQ ID No. 38), and **01_B01** (consisting of the heavy chain amino acid sequence of SEQ ID No. 39 and the light chain amino acid sequence of SEQ ID No. 40).

According to the present invention, the light and heavy chain variable region sequences of the preferred antibodies and antigen-binding fragments thereof described herein with the internal designations 04_A06, 01_D03, 04_B09, 05_C07 and 01_B01 are as follows:

| **SEQ ID NO** | **Source** | **Sequence** |
|---|---|---|
| 31 | Heavy chain variable domain 04_A06 | SVRLDQSGTAVKKPGASVRVSCRAPDSFTVYRPRLSAYFIGE FNIHWLRQAPGQGLEWLGFVNIFRGAVKYSSRFQGRITITRDT SSETSYLDLGALKADDTATYYCAWDKNVDDNPWRLDSWGQ GTLVIVSS |
| 32 | Light chain variable domain 04_A06 | YIQVTQSPSSLSASIGDTITVACEVSQDVGWAVNWYHQRPGR PPYNLIYTAHNLAPGVASRFRGSRVGTYFTLTINNLLPEDVGT YYCQVFDSFAPGGTRVDL |
| 33 | Heavy chain variable domain 01_D03 | HVQLWQSGAEVKKPGASVKISCSAWGFGTTSGYSFRDYRVH WVRHIAGQGFQWMGHMQPRYGAVNYARQFQGRITMTREVS FDASGGTAYMELRSLRSDDTAVYYCVTHKLYDGEDASDLTW RLDPWGQGTRVIVSS |
| 34 | Light chain variable domain 01_D03 | GVHLTQSPASLSASVGDRVTFTCQASQDITNAINWYQLRPGK TPKLMIHDGSTLRRGVPSRFAGSGFGTKFTFTIDNLQPEDLAT YFCQHYEFFPPVGTQVELN |
| 35 | Heavy chain variable domain 04_B09 | SVRLDQSGTAVKKPGASVRVSCRAPDSFTVYRPRLSAYFIGE FNIHWLRQAPGQGLEWLGFVNIFRGAVKYSSAFRGRITITRDT SLETSYLDLGALKPDDTATYFCAWDKNVDDNPWRLDSWGQG TLVVVSS |
| 36 | Light chain variable domain 04_B09 | YIQVTQSPSSLSASVGDTITVACQVSQDVGWAVNWYHQRPG RPPYNLIYTTSNLAPGVASRFRGTRVATYFTLTINNLLPEDVGT YYCQVFESFAPGGTRVDLR |
| 37 | Heavy chain variable domain 05_C07 | HVQLWQSGAEVKKPGASVKISCSAWGFGTRSGYTFRDYRIH WVRQVPRQGFEWMGHLHPAYGAVNYARQFQGRIIMTREVSF DGSGGTVYMELRSLRSDDTATYYCVTHKLYDGEDASDLTWR LDPWGQGTRVTVSS |
| 38 | Light chain variable domain 05_C07 | GVSLTQSPASLCASVGDRVTFTCQASQDITNAINWYQLRPGK TPKLMIHDGSTLRRGVPSRFAGSGFGTRFTFTIDNLQPEDLAT YFCQHYEFFPPAGTQVDFN |
| 39 | Heavy chain variable domain 01_B01 | HVQLWQSGAEVKKPGASVKISCAAWGFGTTSGYSFRDYRIH WVRQVPGQGFQWMGHIQPRYGAVNYAHQFQGRITMTREVS FDASGGTAYMELRSLRSDDTATYYCVTHKLYDGEDASDLAW RLDPWGQGTRVTVSS |
| 40 | Light chain variable domain 01_B01 | GVHLTQSPASLSASVGDRVTFTCQASQDITNAINWYQLRPGK TPKLMIHDGSTLRRGVPSRFAGGGFGTKFTFTIDNLQPEDLAT YFCQHYEFFPPAGTQVELN |

According to one embodiment of the present invention, the antibody or antigen-binding fragment thereof comprises a heavy chain variable region amino acid sequence of antibody 04_A06 (SEQ ID No. 31). According to one embodiment of the present invention, the antibody or antigen-binding fragment thereof comprises a heavy chain variable region amino acid sequence of antibody 01_D03 (SEQ ID No. 33). According to one embodiment of the present invention, the antibody or antigen-binding fragment thereof comprises a heavy chain variable region amino acid sequence of antibody 04_B09 (SEQ ID No. 35). According to one embodiment of the present invention, the antibody or antigen-binding fragment thereof comprises a heavy chain variable region amino acid sequence of antibody 05_C07 (SEQ ID No. 37). According to an embodiment of the present invention, the antibody or antigen-binding fragment thereof comprises a heavy chain variable region amino acid sequence of antibody 01_B01 (SEQ ID No. 39).

According to an embodiment of the present invention, the antibody or antigen-binding fragment thereof comprises a light chain variable region amino acid sequence of antibody 04_A06 (SEQ ID No. 32). According to an embodiment of the present invention, the antibody or antigen-binding fragment thereof comprises a light chain variable region amino acid sequence of antibody 01_D03 (SEQ ID No. 34). According to an embodiment of the present invention, the antibody or antigen-binding fragment thereof comprises a light chain variable region amino acid sequence of antibody 04_B09 (SEQ ID No. 36). According to an embodiment of the present invention, the antibody or antigen-binding fragment thereof comprises a light chain variable region amino acid sequence of antibody 05_C07 (SEQ ID No. 38). According to an embodiment of the present invention, the antibody or antigen-binding fragment thereof comprises a light chain variable region amino acid sequence of antibody 01_B01 (SEQ ID No. 40).

According to a preferred embodiment of the present invention, the antibody or antigen-binding fragment comprises a heavy chain variable region amino acid sequence of SEQ ID No. 31 and a light chain variable region amino acid sequence of SEQ ID No. 32. According to a preferred embodiment of the present invention, the antibody or antigen-binding fragment comprises a heavy chain variable region amino acid sequence of SEQ ID No. 33 and a light chain variable region amino acid sequence of SEQ ID No. 34. According to a preferred embodiment of the present invention, the antibody or antigen-binding fragment comprises a heavy chain variable region amino acid sequence of SEQ ID No. 35 and a light chain variable region amino acid sequence of SEQ ID No. 36. According to a preferred embodiment of the present invention, the antibody or antigen-binding fragment comprises a heavy chain variable region amino acid sequence of SEQ ID No. 37 and a light chain variable region amino acid sequence of SEQ ID No. 38. According to a preferred embodiment of the present invention, the antibody or antigen-binding fragment comprises a heavy chain variable region amino acid sequence of SEQ ID No. 39 and a light chain variable region amino acid sequence of SEQ ID No. 40.

According to a specific embodiment of the present invention, the antibody consists of two heavy chains of sequence SEQ ID No. 31 and two light chains of sequence SEQ ID No. 32, or the antibody consists of two heavy chains of sequence SEQ ID No. 33 and two light chains of sequence SEQ ID No. 34, or the antibody consists of two heavy chains of sequence SEQ ID No. 35 and two light chains of sequence SEQ ID No. 36, or the antibody consists of two heavy chains of sequence SEQ ID No. 37 and two light chains of sequence SEQ ID No. 38, or the antibody consists of two heavy chains of sequence SEQ ID No. 39 and two light chains of sequence SEQ ID No. 40.

According to a preferred embodiment of the present invention, the antibody used as a source for sequences comprised in the antibody or antigen-binding fragment thereof according to the present invention is selected from the group comprising 04_A06, 01_D03, 04_B09 and 05_C07, preferably from the group comprising 04_A06, 01_D03, and 04_B09, more preferably from the group comprising 04_A06 and 01_D03, particularly preferably is the antibody 04_A06.

In one embodiment of the present invention, the antibody used as a source for sequences comprised in the antibody of the invention is 04_A06. In another embodiment of the present invention, the antibody used as a source for sequences comprised in the antibody of the invention is 01_D03. In one embodiment of the present invention, the antibody used as a source for sequences comprised in the antibody of the invention is 04_B09. In another embodiment of the present invention, the antibody used as a source for sequences comprised in the antibody of the invention is 05_C07. In another embodiment of the present invention, the antibody used as a source for sequences comprised in the antibody of the invention is 01_B01.
In one preferred embodiment, the antibody is a monoclonal antibody or a fragment thereof that retains binding specificity and ability to neutralize infectious pathogen. In one preferred embodiment, the antibody is an IgG1, IgG2, IgG3, or IgG4 antibody. For example, the antibody may be an antibody comprising an Fc domain of any human IgG isotype (e.g. IgG1, IgG2, IgG3, or IgG4).

Optionally, the antigen-binding compound consists of or comprises a Fab, Fab', Fab'-SH, F(ab)₂, Fv, a diabody, single-chain antibody fragment, or a multispecific antibody comprising multiple different antibody fragments.

Within the present invention, an antibody or binding fragment directed against the CD4 binding site of HIV-1 means an antibody binding to the CD4 binding site region within the gp120 envelope glycoprotein of HIV-1 with an at least 10-fold, more preferably at least 50-fold, particularly preferably at least 100-fold increased affinity compared to unrelated epitopes, proteins or protein regions. In general, the term CD4 binding site herein designates the CD4 binding site region within the gp120 envelope glycoprotein of HIV-1.

The present invention further provides and encompasses monoclonal human antibodies or binding fragments which comprise antibody amino acid sequences being at least 80% identical to the sequences as defined and disclosed herein as long as they are still directed against the CD4 binding site of the human immunodeficiency virus HIV-1. This is meant to include sequences having trivial mutations of the antibody amino acid sequence which do not interfere with structural folds and the affinity of the antibody to the CD4 binding site.

Commonly and preferably, such mutations which do not substantially interfere with the structure and function of the antibody lie outside of the CDRs of the antibody, more preferably such mutations lie outside the CDRs and outside the framework regions of the antibody. Accordingly, it is preferred that the antibody amino acid sequences which are at least 80% identical to the sequences as defined herein do not carry mutations in the CDRs, more preferably not in the CDRs or in the framework regions of the antibody, if any mutations are present at all.

It is a trivial task for a skilled person to determine if an antibody which exhibits a certain degree of identity is directed against the CD4 binding site of the human immunodeficiency virus HIV-1 based on the above or the common general knowledge.

The determination of percent identity between two sequences is accomplished according to the present invention by using the mathematical algorithm of Karlin and Altschul (Proc. Natl. Acad. Sci. USA (1993) 90: 5873-5877). Such an algorithm is the basis of the BLASTN and BLASTP programs of Altschul et al. (J. Mol. Biol. (1990) 215: 403-410). BLAST nucleotide searches are performed with the BLASTN program. To obtain gapped alignments for comparative purposes, Gapped BLAST is utilized as described by Altschul et al. (Nucleic Acids Res. (1997) 25: 3389-3402). When utilizing BLAST and Gapped BLAST programs, the default parameters of the respective programs are used.

According to a preferred embodiment of the present invention, antibody amino acid sequences form part of the invention which consist of or comprise a nucleic acid sequence being at least 85% identical to the sequences defined above and disclosed herein, more preferably at least 90% identical, even more preferred at least 95% identical. According to one preferred embodiment, the mutations in the amino acid sequence which may lead to a sequence identity of less than 100% lie outside of the CDRs disclosed herein, more preferably such mutations lie outside the CDR and the framework regions of the antibody sequences disclosed herein.

Within the present invention, the antibodies disclosed are tested for their neutralizing activity against a wide selection of pseudoviruses. The TZM-bl cell pseudovirus neutralization assay used to study the breadth and strength of neutralization is a highly standardized assay which is commonly used in the art and in the technical field of the invention for an analysis of neutralization potency of antibodies against various HIV-1 strains. In brief, antibodies and virus strains are incubated together before TZM-bl target cells are added. These cells exhibit luciferase activity in case of a successful infection, resulting in a detectable luminescence signal in the presence of luciferin after lysis of cells. Neutralizing antibodies are able to prevent infection and therefore the generation of luminescence. The potency of a neutralizing antibody is determined by the concentration of the antibody required to reduce virus infectivity by a particular amount.

An exemplary and standardized way of setting up and employing this assay is disclosed and described in detail in Sarzotti-Kelsoe et al.; J Immunol Methods. 2014 July ; 0: 131-146. doi:10.1016/j.jim.2013.11.022. Preferably, the method described in the "Examples" section below is used to determine the neutralizing activity. This description alone and in combination with the common prior art enables the skilled person to establish and determine a conclusive readout of the TZM-bl cell pseudovirus neutralization assay as used herein.

Neutralization potency as defined herein preferably takes only those virus variants into account that could positively be determined as being neutralized by the respective antibodies. Based on the selection of positively neutralized variants, a geometric mean IC₅₀ is then determined across the neutralized strains.

According to a preferred embodiment of the first aspect of the present invention, the antibody or antigen-binding fragment thereof comprises a combination of the variable region heavy chain amino acid sequence and of the variable region light chain amino acid sequence of the antibody 04_A06 (consisting of the heavy chain amino acid sequence of SEQ ID No. 31 and the light chain amino acid sequence of SEQ ID No. 32).

Preferably, the antibodies or antigen-binding fragments as described herein are mammalian, more preferably human. Further preferably, the antibodies or antigen-binding fragments as described herein are monoclonal, and may be used and administered in a monoclonal fashion, i.e. without a concurrent or overlapping administration of another monoclonal antibody to a patient. Alternatively, preferably, the antibodies or antigen-binding fragments of the present invention may be administered in combination with one or more different monoclonal antibodies, e.g. in a concurrent or overlapping administration, so that all of said antibodies deliver their effects concomitantly.

In the description of the present application, antibody designations may be used. It is pointed out that the antibodies consist of heavy and light chains which also form part of the present description. If reference is made to an antibody by its designation or to a SEQ ID No., it should be understood that these ways of reference are interchangeable.

The present invention further relates in a second aspect to a pharmaceutical composition comprising an antibody or antigen-binding fragment thereof according to the first aspect of the present invention, and at least one pharmaceutically acceptable excipient. Preferably, the pharmaceutical composition according to the second aspect of the present invention is a vaccination composition for a human subject.

Preferably, the pharmaceutical composition according to the second aspect of the present invention may further comprise one or more antibodies directed against a different epitope within HIV-1, preferably directed against the HIV-1 envelope (Env) trimer, more preferably directed against the V3 glycan site.

The present invention also encompasses in a third aspect a kit comprising an antibody or antigen-binding fragment thereof according to the first aspect of the present invention and a container.

In a fourth aspect of the present invention, the antibody or antigen-binding fragment thereof according to the first aspect of the present invention, the pharmaceutical composition according to the second aspect, and the kit according to the third aspect of the present invention are for use as a medicament.

In a fifth aspect of the present invention, the antibody or antigen-binding fragment thereof according to the first aspect of the present invention, the pharmaceutical composition according to the second aspect, and the kit according to the third aspect of the present invention are for use in the treatment or prevention of an infection with the human immunodeficiency virus HIV-1 in mammalian subjects.

According to a sixth aspect of the present invention, the antibody or antigen-binding fragment thereof according to the first aspect of the present invention, the pharmaceutical composition according to the second aspect, and the kit according to the third aspect of the present invention are for use in the treatment or prevention of a disease caused by the human immunodeficiency virus HIV-1 in mammalian subjects.

In a preferred embodiment of the fifth or sixth aspect of the present invention, the use is in human subjects. In another preferred embodiment of the fifth or sixth aspect of the present invention, the use is for vaccination and/or in the form of a vaccination composition. In yet another preferred embodiment of the fifth or sixth aspect of the present invention, the use is in in the treatment or prevention of acquired immune deficiency syndrome (AIDS).

In one other aspect, the present invention is also directed to a method of treatment of a patient suffering from a disease caused by the human immunodeficiency virus HIV-1 in human subjects, preferably for use in the treatment or prevention of acquired immune deficiency syndrome (AIDS) in human subjects, wherein the patient is administered an effective amount of the antibody or antigen-binding fragment thereof according to the invention or a pharmaceutical composition of the invention.

In another aspect, the present invention is also directed to the use of the antibody or antigen-binding fragment thereof according to the invention or a pharmaceutical composition of the invention in the manufacture of a medicament for treatment of a disease caused by the human immunodeficiency virus HIV-1 in human subjects, preferably for treatment or prevention of acquired immune deficiency syndrome (AIDS) in human subjects.

According to the seventh aspect of the present invention, a nucleic acid is provided encoding an antibody or antigen-binding fragment thereof according to the first aspect of the present invention.

According to the eighth aspect of the present invention, an expression vector comprising the nucleic acid of the seventh aspect of the present invention is provided in functional association with an expression control sequence.

According to the ninth aspect of the present invention, a host cell is provided comprising a nucleic acid according to the seventh aspect of the present invention or the expression vector according to the eighth aspect of the present invention.

According to the tenth aspect of the present invention, a method of production of an antibody or antigen-binding fragment thereof according to the first aspect of the present invention is provided, comprising cultivating the host cell of the ninth aspect of the present invention under conditions allowing expression of the antibody or antigen-binding fragment thereof, and recovering the antibody or antigen-binding fragment thereof.

All embodiments of the present invention as described herein are deemed to be combinable in any combination, unless the skilled person considers such a combination to not make any technical sense.

### Examples

### A) Experimental Methods

### Isolation of Monoclonal Antibody Sequences

Blood and leukapheresis samples were obtained under protocols approved by the Institutional Review Board of the University of Cologne (protocols 13-364 and 16-054) and the participant provided written informed consent. Peripheral blood mononuclear cells (PBMCs) were isolated by density-gradient centrifugation and stored at -150°C in 90% FBS and 10% DMSO. B cells were isolated from PBMCs by magnetic cell separation and labeled with anti-human CD19-AF700, anti-human IgG-APC, DAPI (BD), and an HIV-1 Env bait protein for 30 minutes on ice.

The HIV-1 Env bait protein was either BG505_{SOSIP.664}-GFP (Sliepen K et al., Biomolecules, 2015 Oct 23;5(4):2919-34) or biotinylated (EZ-Link Sulfo NHS Bioting and Labeling Kit, Thermo Fisher) YU2_{gp140} (Yang et al., J Virol. 2000 Jun;74(12):5716-25. doi: 10.1128/jvi.74.12.5716-5725.2000) that was labeled with Streptavidin-PE. Env-reactive CD19⁺IgG⁺DAPI⁻ single cells were sorted as described before (Gieselmann et al., Nat Protoc. 2021 Jul;16(7):3639-3671. doi: 10.1038/s41596-021-00554-w.). Sorted cells were incubated with random hexamer primer, NP-40, and RNase-free H₂O for 1 min at 65°C. Subsequently, cDNA was generated using SuperScript IV in the presence of RT Buffer, dNTPs, DTT, H₂O, RNasin, and RNaseOUT. Antibody sequences for single cell analysis were amplified by semi-nested PCRs using Taq polymerase and the previously described primers CG_RT (Ozawa et al., Biotechniques. 2006 Apr;40(4):469-70, 472, 474 passim. doi: 10.2144/000112123, first PCR), IgG_Internal RT (Tiller et al., J Immunol Methods. 2008 Jan 1;329(1-2):112-24. doi: 10.1016/j.jim.2007.09.017. Epub 2007 Oct 31. , second PCR), and the OPT5/oPR-primer mix (Kreer et al., J Immunol Methods. 2020 May;480:112752. doi: 10.1016/j.jim.2020.112752, both PCRs)

### Antibody Sequence Analysis

Sequences of 2nd PCR products with a mean Phred score ≥28 and a minimal length of 240 nucleotides were annotated with IgBLAST (Ye et al., 2013) and trimmed from framework region (FWR) 1 of the variable region to the end of the J gene. Base calls with a Phred score <16 were masked and sequences with >15 masked nucleotides, frameshifts, or stop codons were excluded from further analyses. To analyze the sequences for potential clonalities, all productive heavy chain sequences were grouped by identical V genes and the pairwise Levenshtein distance of their CDRH3s was determined.

Individual sequences were grouped into clones when they shared the same V gene and had a minimal CDRH3 identity of 75%. After 10 rounds with a randomized input of sequences the result that yielded the lowest number of unassigned (non-clonal) sequences was selected for further analyses. All clones were re-validated manually by the investigators in order to identify shared mutations. Sequences that were initially assigned to different clones but shared the same VDJ genes and amino acid and/or silent nucleotide mutations were subsequently grouped into subclones. Nucleotide sequence identity to germline was calculated using IgBLAST.

### Monoclonal Antibody Production

For cloning of single cell-derived antibodies, the 1st PCR product of single cell-PCR was used as template and amplified using Q5 High Fidelity Polymerase and specific forward- and reverse primers that resembled the respective nucleotide sequence of the V- and J-regions (Gieselmann et al., 2021, *supra*) with expression vector overhangs for subsequent sequence and ligation independent cloning (SLIC). PCR products were cloned into human antibody expression vectors (lgG1, kappa, or lambda chain) by SLIC assembly as previously described (von Boehmer et al., Nat Protoc. 2016 Oct; 11(10):1908-1923. doi: 10.1038/nprot.2016.102). Antibodies were produced in HEK293-6E cells by transfection using polyethylenimine. After 5-7 days, antibodies were purified from supernatants after protein G incubation and subsequent elution from chromatography columns using 0.1 M glycine (pH=3.0). After buffer neutralization, buffer exchange to PBS, and filter-sterilization, antibodies were stored at 4°C.

### Pseudovirus Production

Pseudoviruses were produced in HEK293T cells by co-transfection with pSG3ΔEnv plasmid as described previously (Doria-Rose et al., PLoS Pathog. 2017 Jan 4;13(1):e1006148. doi: 10.1371/journal.ppat; Sarzotti-Kelsoe et al., J Immunol Methods. 2014 Jul;409:131-46. doi: 10.1016/j.jim.2013.11.022; Hraber et al., J Virol. 2017 Sep 12;91(19):e00991-17. doi: 10.1128/JVI.00991-17.; Seaman et al., J Virol. 2010 Feb;84(3):1439-52. doi: 10.1128/JVI .02108-09).

### TZM-bl Cell Neutralization Assay

Neutralization assays were performed as previously described (Sarzotti-Kelsoe et al., 2014, *supra*; Seaman et al., 2010, *supra*)*.* Murine leukemia virus (MuLV)-pseudotyped virus was used to determine unspecific activity. Antibodies were tested in duplicates. For the global reference panel, bioluminescence was determined after adding a luciferin/lysis-buffer (10 mM MgCl2, 0.3 mM ATP, 0.5mM Coenzyme A, 17 mM IGEPAL (all Sigma-Aldrich), and 1mM D-Luciferin (GoldBio) in Tris-HCL).

### HIV-1-infected Humanized Mice

Humanized mice were generated as previously described (Klein et al., 2012) with modifications. NOD.Cg-*Rag1^{tm1mom}II2rg^{tm1Wjl}*/SzJ (NRG) mice were humanized within five days of birth and 3-6 hours after sublethal irradiation by intrahepatic injection of human CD34⁺ hematopoietic cord blood and/or placental tissue stem cells. Humanized mice were infected by intraperitoneal challenge using replication-competent recombinant HIV-1_{YU2} (YU2 *env* in NL4-3 backbone (Zhang et al., J Virol. 2002 Jun;76(12):6332-43. doi: 10.1128/jvi.76.12.6332-6343.2002) that was harvested from supernatants of transfected HEK293T cells.

### HIV-1 Viral Load Measurements

Plasma RNA was extracted from EDTA plasma samples using the MinElute Virus Mini Spin Kit including an on-column DNase I digestion step. Viral loads were determined by quantitative real-time PCR using *pol*-specific primers described previously (Horwitz et al., Proc Natl Acad Sci USA. 2013 Oct 8;110(41):16538-43. doi: 10.1073/pnas.1315295110). qPCR was performed on a QuantStudio 5 using the Taqman RNA-to-Ct 1-Step-Kit. Viral loads were quantified by including a standard curve derived from a sample of known copy number with every qPCR run. The limit of accuracy of the qPCR was determined as 784 copies/ml.

### Antibody Treatment of HIV-1-infected Humanized Mice

Antibodies diluted in PBS were injected subcutaneously. Following a 1 mg loading dose, doses of 0.5 mg were injected every 3-4 days.

### B) Specific Examples of Antibodies of the Invention

### Example I - Isolation of Broad and Potent VH1-2 Derived HIV-1-Neutralizing Antibodies from an HIV-1-Infected Elite Neutralizer

Human B cells reactive to the HIV-1 envelope protein were isolated from an HIV-1-infected individual who was previously identified as having exceptional serum neutralizing activity against HIV-1 in *in vitro* assays.

To this end, isolated B cells were incubated with fluorochrome-labeled soluble HIV-1 Env protein (YU2_{gp140} or BG505_{SOSIP.664}) and single-cell sorted (Gieselmann et al., 2021, *supra*)*.* This approach enabled the subsequent amplification of heavy and light antibody gene segments from an individual HIV-1 Env-reactive B cell by PCR. This allowed for the cloning of the PCR products into expression vectors for the recombinant production of the corresponding antibody encoded by an individual B cell, enabling functional testing of the antibody.

From the analysis of the sequences of HIV-1-Env reactive B cells, expanded VH1-2-derived B cell clones could be identified. One of these clones was characterized by a twelve amino acid (aa) insertion and a deletion of one amino acid in heavy chain CDR1/FWR1 region. Antibody 04_A06 is representative of this B cell clone. Another of these clones, encompasses a 6 aa CDRH1/FWRH1 and a 4 aa FWRH3 insertion. Antibody 01_D03 is a representative of this B cell clone.

To determine the overall neutralizing potency and breadth, isolated antibody 04_A06 as well as other antibodies were tested for neutralizing activity in the TZM-bl cell neutralization assay against a reference panel of 12 HIV-1 pseudovirus strains that is referred to as "global panel" (**Figure 1**). This panel of pseudoviruses was previously designed to be a representation of the diversity of the global HIV-1 epidemic and enable a standardized assessment of the activity of neutralizing antibodies.

Notably, all antibodies of the present invention (i.e. 04_A06, 01_D03, 04_B09, 05_C07 and 01_B01) demonstrated high neutralizing activity against 100% (12/12) of the strains included in the panel (**Figure 1****)**. The neutralizing potency is routinely represented by the 50% inhibitory concentration (IC₅₀). When tested against the "global panel", all of the antibodies of the present invention exhibited a geometric mean IC₅₀ of less than 0.05 µg/ml, wherein 04_A06 and 01_D03 were highly active with a geometric mean IC₅₀ of 0.038 µg/ml or 0.021 µg/ml against the neutralized virus strains (**Figure 1**).

### Example II - High Potency and Breadth of Antibody 04_A06

To confirm the neutralizing potency and breadth of 04_A06, the antibody was tested against an extended panel of 119 HIV-1 pseudoviruses as previously tested in Schoofs et al., 2019. This panel of pseudoviruses represents a development of a panel of pseudoviruses described in detail in Seaman et al., 2010. It provides a representation of the genetic and global diversity of HIV-1 Env variants. It includes HIV-1 variants of different clades or subtypes, including variants isolated from transmitted/founder viruses and difficult-to-neutralize viruses.

Notably, 04_A06 demonstrated high neutralizing potency and breadth when tested on the 119-pseudovirus multiclade panel (**Figure 2**). In particular, when tested at antibody concentrations up to 10 µg/ml, antibody 04_A06 neutralized 99% of the tested pseudoviruses and showed a geometric mean IC₅₀ of 0.036 µg/ml against the neutralized pseudoviruses (**Figure 2**). This breadth and potency are higher than seen for other HIV-1 neutralizing antibodies that also target the CD4 binding site and are in advanced stages of clinical testing (e.g. 3BNC117, VRC01, and VRC07_{-523-LS}) (**Figure 2**). Moreover, the potency of antibody 04_A06 in the 119-multiclade panel was higher than that of N6, another VH1-2-derived CD4 binding site antibody that is currently investigated in clinical testing (**Figure 2**).

In addition, when compared to other HIV-1 neutralizing antibodies and other CD4bs bNAbs, for which results against a total of 76 (**Figure 3** **A**) and 99 (**Figure 3** **B**) identical HIV-1 pseudovirus were available (Yoon et al., 2015, *supra*)*,* 04_A06 demonstrated the highest levels of potency and/or breadth.

### Example III- High Potency and Breadth of Antibody 04_A06 against Clade C HIV-1 Pseudoviruses

HIV-1 viruses of the group M can be divided into nine clades and different recombinants. HIV-1 viruses from clade C are responsible for approximately half of current HIV-1 infections and represent the most common lineage worldwide. Therefore, clade C viruses are investigated in many different antibody and vaccine studies. In order to confirm the antiviral activity of antibody 04_A06 against clinically relevant HIV-1 clades, we determined the neutralization profile of the antibody against a published reference panel, that consists of 100 HIV-1 pseudoviruses isolated from different stages of infection and difficult-to-neutralize viruses. The so-called Subtype C panel is described in detail in Hraber et al., J Virol., 2017 Sep 12;91(19):e00991-17.

Notably, 04_A06 demonstrated high neutralizing potency and breadth when tested against the 100 strain Subtype C pseudovirus panel (**Figure 4**). In particular, when tested at antibody concentrations up to 10 µg/ml, antibody 04_A06 neutralized 99% of the tested pseudoviruses and showed a geometric mean IC₅₀ of 0.057 µg/ml against the neutralized pseudoviruses (**Figure 4**). The breadth and potency of 04_A06 is higher than observed for the best CD4bs bNAbs investigated against the Subtype C pseudovirus panel so far (3BNC117, 1-18, N6, VRC01, VRC07_{-523-LS}) (**Figure 4**). Neutralization data for the CD4bs reference bNAbs was obtained from the CATNAP database (Yoon et al., 2015, *supra*)*.*

### Example IV - Neutralizing Activity against Pseudoviruses that are Poorly Neutralized by other CD4 binding site antibodies

Although CD4 binding site antibodies can achieve high levels of neutralizing breadth (i.e., have activity against a large number of different HIV-1 Env variants), antibody-resistant HIV-1 variants exist. Thus, it is important to identify novel CD4 binding site antibodies that are highly active against such HIV-1 variants. Therefore, we determined the neutralization profile of antibody 04_A06 described in this invention against a panel of nine (**Figure 5**) and seven (**Figure 6****)** HIV-1 variants that are resistant against neutralization by CD4bs bNAbs.

Tested virus strains illustrated in **Figure 5** are constituents of the 119 multiclade and Subtype C pseudovirus panel (as described in as described in Schoofs et al., Immunity, 2019 Jun 18;50(6):1513-1529.e9, Hraber et al., J Virol., 2017 Sep 12;91(19):e00991-17). Tested virus strains illustrated in **Figure 6** were selected from a reference pseudovirus panel that is known as "Resistant panel to CD4bs bNAbs" and is described in Zhou et al., PLoS Pathog. 2019 Jun 13;15(6):e1007819. Doi: 10.1371/journal.ppat.1007819. PMID: 31194843; PMCID: PMC6592578 2017 Sep 12;91(19):e00991-17.

Compared to CD4 binding site antibodies in advanced stages of clinical testing and to previously published best in class CD4bs bNAbs (VRC07_{-523-LS}, N6, 3BNC117, 1-18, VRC01), antibody 04_A06 neutralizes resistant pseudovirus variants with much higher breadth and/or potency (**Figure 5** and **Figure 6**).

For example, 100% of the nine pseudovirus panel and 86% of the seven pseudovirus panel are neutralized by 04_A06 when tested at concentrations up to 10 µg/ml in the TZM-bl pseudovirus neutralization assay. In contrast, the broadest and most potent CD4bs reference bNAbs described so far only neutralize between 0% - 67% of the nine pseudovirus panel (**Figure 5**) and 0% - 57% of the seven pseudovirus panel (**Figure 6**). In addition, 04_A06 displays the highest potency (IC₅₀ of 0.153 µg/ml) against strains of the seven pseudovirus panel compared to reference CD4bs bNAbs (**Figure 6**).

Thus, antibodies of this invention provide a solution to the poor neutralization of viruses that are resistant against neutralization by CD4bs bNAbs.

### Example V - Neutralizing Activity of Antibody 01_D03 Against Pseudoviruses that are Insensitive to Neutralization by CD4bs bNAbs

In addition to virus variants that are resistant, there are also variants that are insensitive against neutralization by most CD4bs bNAbs. Clinical trials have shown that the sensitivity of virus variants critically determine the success of bNAb-mediated treatment or prevention strategies. The inventors investigated the neutralization profile of antibody 01_D03 against a virus panel that consists of eight pseudovirus strains (**Figure 7**) which are known to be insensitive against neutralization by most CD4bs bNAbs.

Viral strains of this panel are constituents of the 119 multiclade pseudovirus panel (as described in Schoofs et al., Immunity, 2019 Jun 18;50(6):1513-1529.e9). Insensitivity against neutralization by a given antibody was defined by an IC₅₀ > 1 µg/ml. Antibody 01_D03 neutralizes 88% (7/8) of the viral strains of the eight pseudovirus panel whereas the broadest and most potent reference bNAbs against CD4bs described so far only neutralize 25% - 75% (**Figure 7**). Thus, antibodies of this invention provide a solution to the poor neutralization of viruses that are insensitive against neutralization by most other CD4bs bNAbs.

### Example VI - Neutralizing Activity against Pseudoviruses generated from the Antibody Mediated Prevention Trials

The recent Antibody Mediated Prevention (AMP) Trials have provided proof of concept that bNAb administration can in principle be effective to prevent HIV-1 infections in humans. In this trial, the prevention efficacy of CD4bs bNAb VRC01 strongly depended on the sensitivity of circulating viral strains against the antibody. VRC01 displayed a prevention efficacy of 75% against virus strains that were neutralized with an ICso below 1 µg/ml (as described in Corey L, Two Randomized Trials of Neutralizing Antibodies to Prevent HIV-1 Acquisition. N Engl J Med. 2021 Mar 18;384(11):1003-1014. Doi: 10.1056/NEJMoa2031738. PMID: 33730454; PMCID: PMC8189692).

Antibody 04_A06 neutralized up to 98% of 185 pseudovirus strains generated from the virus sequences obtained from the AMP trials (**Figure 8**). In contrast, VRC01 only displayed a breadth of up to 83% against the tested pseudovirus panel. In addition, the GeoMean ICso (0.084 µg/ml) of 04_A06 against the virus panel was approximately 10-fold higher than for VRC01 (0.839 µg/ml). Thus, 04_A06 can be regarded as a promising bNAb candidate for future antibody-based prevention strategies.

### Example VII- Maintaining Viral Suppression by Antibody Monotherapy in an In Vivo Model of HIV-1-Infection

Humanized mice infected with HIV-1_{YU2} (Zhang et al., 2002, *supra*) provide a model to study the antiviral activity of neutralizing HIV-1 antibodies *in vivo.* To generate humanized mice, immunodeficient NRG mice are irradiated within the first days after birth and injected intrahepatically with human hematopoietic CD34⁺ stem cells. This results in the development of human lymphocytes that can be infected with replication-competent HIV-1. These mice can maintain stable levels of viremia (i.e., HIV-1 RNA copy numbers in plasma) and show a rate of HIV-1 sequence diversification that is similar to what is observed in humans (Klein et al., Nature. 2012 Dec 6;492(7427):118-22. doi: 10.1038/nature11604). This mouse model has previously been used to determine the antiviral activity of several CD4 binding site antibodies given as monotherapy *in vivo* (179NC75: Freund et al., PLoS Pathog. 2015 Oct 30;11(10):e1005238. doi: 10.1371/journal.ppat.1005238; 3BNC117: Horwitz et al, Proc Natl Acad Sci USA. 2013 Oct 8;110(41):16538-43. doi: 10.1073/pnas.1315295110; 45-46^{G54W}: Klein et al, 2012, *supra*; NC37: Freund et al., Sci Transl Med. 2017 Jan 18;9(373):eaal2144. doi: 10.1126/scitranslmed.aal2144).

In all of these studies, only transient effects on the viral load were observed and viral rebound (i.e., return of viremia towards baseline levels) rapidly developed. Moreover, this viral rebound was associated with the development of mutations in the antibody target sites. Thus, novel CD4 binding site antibodies are required that are capable of maintaining viral suppression when given as monotherapy.

When HIV-1_{YU2}-infected humanized mice were treated with the CD4 binding site antibody VRC01 or VRC07 (1 mg loading dose per antibody subcutaneously (s.c.), followed by 0.5 mg given s.c. every 3-4 days), only a transient reduction of the HIV-1 RNA copy number was observed, and viremia returned to baseline levels in most mice within 2-4 weeks (**Figure 9**). In contrast, when mice were treated according to the same dosing scheme with the representative antibody 04_A06 of the invention, viral suppression compared to the viral copy number at the start of treatment was observed in all treated mice for a period of 84 days of treatment (**Figure 9**). In all mice (100%), the HIV-1 RNA plasma copy number was reduced to levels below the limit of accuracy of the used assay (784 copies/ml).

Thus, 04_A06 is capable to effectively induce and maintain viral suppression in HIV-1_{YU2}-infected humanized mice even when given as monotherapy.

### Example VIII - Maintaining Viral Suppression by Antibody Monotherapy in an In Vivo Model of HIV-1-Infection After Viral Rebound from VRC01-Class Therapy

Failure of antibody monotherapy using CD4 binding site antibodies in HIV-1-infected humanized mice results in viral rebound and is associated with viral resistance against the administered antibody (Freund et al., 2015, *supra*; Horwitz et al, 2013, *supra*; Klein et al, 2012, *supra*; Freund et al., 2017, *supra*)*.* Thus, novel CD4 binding site antibodies are required that provide an *in vivo* treatment option after previous failure of CD4 binding site therapy.

Following the viral rebound observed during four weeks of treatment with VRC01 (as described in **Example VII**), the addition of antibody 04_A06 of the invention to treatment regimen (1 mg loading dose s.c. followed by 0.5 mg given s.c. every 3 days to 4 days) resulted in a sustained reduction of viremia in all (100%) of treated mice (**Figure 10**). Thus, antibody 04_A06 provides an option for the *in vivo* control of HIV-1 even after the failure of pretreatment with other CD4 binding site antibodies.

## Claims

1. Antibody or antigen-binding fragment thereof directed against the CD4 binding site of the human immunodeficiency virus HIV-1, wherein the antibody or antigen-binding fragment thereof comprises a combination of the heavy chain CDR1 to CDR3 and the light chain CDR1 to CDR3 amino acid sequence of one antibody selected from the group comprising:
**04_A06** (having a CDR-H1 amino acid sequence of SEQ ID No. 1, a CDR-H2 amino acid sequence of SEQ ID No. 2, a CDR-H3 amino acid sequence of SEQ ID No. 3, a CDR-L1 amino acid sequence of SEQ ID No. 4, a CDR-L2 amino acid sequence of SEQ ID No. 5, and a CDR-L3 amino acid sequence of SEQ ID No. 6),
**01_D03** (having a CDR-H1 amino acid sequence of SEQ ID No. 7, a CDR-H2 amino acid sequence of SEQ ID No. 8, a CDR-H3 amino acid sequence of SEQ ID No. 9, a CDR-L1 amino acid sequence of SEQ ID No. 10, a CDR-L2 amino acid sequence of SEQ ID No. 11, and a CDR-L3 amino acid sequence of SEQ ID No. 12),
**04_B09** (having a CDR-H1 amino acid sequence of SEQ ID No. 13, a CDR-H2 amino acid sequence of SEQ ID No. 14, a CDR-H3 amino acid sequence of SEQ ID No. 15, a CDR-L1 amino acid sequence of SEQ ID No. 16, a CDR-L2 amino acid sequence of SEQ ID No. 17, and a CDR-L3 amino acid sequence of SEQ ID No. 18),
**05_C07** (having a CDR-H1 amino acid sequence of SEQ ID No. 19, a CDR-H2 amino acid sequence of SEQ ID No. 20, a CDR-H3 amino acid sequence of SEQ ID No. 21, a CDR-L1 amino acid sequence of SEQ ID No. 22, a CDR-L2 amino acid sequence of SEQ ID No. 23, and a CDR-L3 amino acid sequence of SEQ ID No. 24), and
**01_B01** (having a CDR-H1 amino acid sequence of SEQ ID No. 25, a CDR-H2 amino acid sequence of SEQ ID No. 26, a CDR-H3 amino acid sequence of SEQ ID No. 27, a CDR-L1 amino acid sequence of SEQ ID No. 28, a CDR-L2 amino acid sequence of SEQ ID No. 29, and a CDR-L3 amino acid sequence of SEQ ID No. 30).

2. Antibody or antigen-binding fragment according to claim 1, wherein the antibody or antigen-binding fragment thereof comprises a combination of the variable region heavy chain amino acid sequence and of the variable region light chain amino acid sequence of one antibody selected from the group comprising:
**04_A06** (consisting of the heavy chain amino acid sequence of SEQ ID No. 31 and the light chain amino acid sequence of SEQ ID No. 32),
**01_D03** (consisting of the heavy chain amino acid sequence of SEQ ID No. 33 and the light chain amino acid sequence of SEQ ID No. 34),
**04_B09** (consisting of the heavy chain amino acid sequence of SEQ ID No. 35 and the light chain amino acid sequence of SEQ ID No. 36),
**05_C07** (consisting of the heavy chain amino acid sequence of SEQ ID No. 37 and the light chain amino acid sequence of SEQ ID No. 38), and
**01_B01** (consisting of the heavy chain amino acid sequence of SEQ ID No. 39 and the light chain amino acid sequence of SEQ ID No. 40).

3. Antibody or antigen-binding fragment thereof according to claim 1 or 2, wherein the amino acid sequences comprised are of one antibody selected from the group comprising 04_A06, 01_D03, 04_B09 and 05_C07, preferably of one antibody from the group comprising 04_A06, 01_D03, and 04_B09, more preferably of one antibody from the group comprising 04_A06 and 01_D03, particularly preferably of the antibody 04_A06.

4. Pharmaceutical composition comprising an antibody or antigen-binding fragment thereof according to any one of claims 1 to 3 and at least one pharmaceutically acceptable excipient.

5. Pharmaceutical composition according to claim 4, wherein the pharmaceutical composition is a vaccination composition for a human subject.

6. Kit comprising an antibody or antigen-binding fragment thereof according to any one of claims 1 to 3 and a container.

7. Antibody or antigen-binding fragment thereof according to any one of claims 1 to 3, pharmaceutical composition according to any one of claims 4 or 5, or kit according to claim 6 for use as a medicament.

8. Antibody or antigen-binding fragment thereof according to any one of claims 1 to 3, pharmaceutical composition according to any one of claims 4 or 5, or kit according to claim 6 for use in the treatment or prevention of an infection with the human immunodeficiency virus HIV-1 in mammalian subjects.

9. Antibody or antigen-binding fragment thereof according to any one of claims 1 to 3, pharmaceutical composition according to any one of claims 4 or 5, or kit according to claim 6 for use in the treatment or prevention of a disease caused by the human immunodeficiency virus HIV-1 in mammalian subjects.

10. Antibody or antigen-binding fragment thereof, pharmaceutical composition, or kit for use according to any one of claims 8 or 9, wherein the use is in human subjects.

11. Antibody or antigen-binding fragment thereof, pharmaceutical composition, or kit for use according to any one of claims 8 to 10, wherein the use is in in the treatment or prevention of acquired immune deficiency syndrome (AIDS).

12. Nucleic acid encoding an antibody or antigen-binding fragment thereof according to any one of claims 1 to 3.

13. An expression vector comprising the nucleic acid of claim 12 in functional association with an expression control sequence.

14. Host cell comprising a nucleic acid according to claim 12 or the expression vector according to claim 13.

15. Method of production of an antibody or antigen-binding fragment thereof according to any one of claims 1 to 3, comprising
(a) cultivating the host cell of claim 14 under conditions allowing expression of the antibody or antigen-binding fragment thereof, and
(b) recovering the antibody or antigen-binding fragment thereof.
